# EUROPEAN PATENT APPLICATION

(11) **EP 4 218 782 A1**
(43) Date of publication of application: **02.08.2023**
(21) Application number: 20955399.9
(22) Date of filing: 18.12.2020
(51) Int. Cl.: A61K 36/185, A61P 13/08, A23L 33/105

(54) **COMPOSITION FOR PREVENTION AND TREATMENT OF BENIGN PROSTATIC HYPERPLASIA, COMPRISING FRUIT EXTRACTS OF ELAEAGNUS MULTRIFLORA THUNB. AS ACTIVE INGREDIENT**

(30) Priority: 24.09.2020 KR 20200123441
(71) Applicant: Jeonnam Bioindustry Foundation, Naju-si, Jeollanam-do 58275 (KR)
(72) Inventor: KIM, Jae Yong, Suncheon-si Jeollanam-do 57970 (KR); LEE, Gyu Ok, Naju-si Jeollanam-do 58275 (KR); YUN, Hyo Jeong, Gwangju 61664 (KR); OH, Kyo Nyeo, Naju-si Jeollanam-do 58275 (KR); CHOI, Chul Yung, Naju-si Jeollanam-do 58275 (KR); LEE, Hak Sung, Naju-si Jeollanam-do 58275 (KR)
(74) Representative: Plougmann Vingtoft a/s
(86) International application number: PCT/KR2020/018651
(87) International publication number: WO 2022/065601

(57) **Abstract**

The present invention relates to a composition for preventing or treating benign prostatic hyperplasia, comprising fruit extracts of Elaeagnus multiflora Thunb.. The fruit extracts of Elaeagnus multiflora Thunb. according to the present invention inhibited the production of 5-alpha reductase-2, prostate-specific antigen (PSA), and 5-alpha-reductase-2 in vitro, and also suppressed the enlargement of prostate tissues and the contents of 5-alpha reductase and prostate-specific antigen (PSA) in a testosterone-induced prostatic hyperplasia rat model, thereby enhancing the excellent effect of preventing or suppressing prostatic hyperplasia even in animal models, and thus have industrial applicability.

## Description

### Technical Field

The present disclosure relates to a composition including an extract from *Elaeagnus multriflora* Thunb. fruits as an active ingredient for prevention and treatment of benign prostatic hyperplasia. More specifically, the present disclosure relates to a pharmaceutical composition for prevention and treatment of benign prostatic hyperplasia, which employs the native material *Elaeagnus multriflora* Thunb. fruit and thus can be safely used with neither toxicity nor adverse effects.

### Background Art

Benign prostatic hyperplasia (BPH) is a type of prostate disease in which the prostate gland around the urethra enlarges due to various causes and presses on the urethra, leading to symptoms, such difficulty to pass urine, etc.

Recently, BPH has been defined as complaints collectively referred to as lower urinary tract symptoms in men, and includes difficulty in passing urine out of the bladder, such as frequent urination (more than 8 times/day), nocturia, urgency (strong and sudden need to urinate, compelling need to urinate that cannot be deferred), urinary hesitancy (a delay between trying to urinate and the flow actually beginning), intermittency (not continuous), need to strain while urinating, etc. It is known that the male hormones testosterone and dihydrotestosterone (DHT) play a permissive role in the development of prostate enlargement.

Medications for BPH are largely divided into alpha blockers and androgen inhibitors (5α-reductase inhibitor). Alpha-blockers are medications that lower pressure and tension in the prostate and urethra, and include terazosin, doxazosin, tamsulosin, and alfuzosin. Common side effects of alpha blockers have been reported to include dizziness, lethargy, headache, and visual disturbance. Androgen inhibitors are medications that prevent prostate enlargement by inhibiting 5-alpha-reductase, and include finasteride and dutasteride. Side effects related to sexual function have been reported.

Elaeagnus multriflora Thunb. is known as a garden tree that is planted in a garden, with the fruit edible. Traditionally in herb medicine, all of its fruits, leaves, stems, and roots have been used as a medicine. Elaeagnus multriflora Thunb. is a plant of the genus Elaeagnus that grows wild in various parts of Korea and is called "ya-ang-do" ( 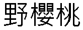 ) or "sa-wol-ja" ( 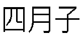 ) in Korean and known as "mock-ban-ha" ( 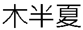 ) in the raw medicinal material field. The tree is commonly called house silverberry and the young branches are covered with reddish-brown scale hairs. It looks like autumn olive in appearance, but with larger fruits and is cultivated in a garden and thus called garden autumn olive. When used as a medicine, Elaeagnus multriflora Thunb. is generally decocted.

The leaves are alternate, 3 to 10 cm long, and oval and have both narrow and sharp ends, with no sawtooth edges. Scale hairs appear on the front side when the plant is young, but gradually disappears, and the back side is characterized by a mixture of white scale hairs and brown scale hairs. Flowers bloom in April-May in light yellow, and one or two flowers hang on the axilla. Flowers have white and brown scale hairs. The calyx tube is rapidly narrowed at the bottom, surrounds the ovary, and splits into 4 ends, with 4 stamens and 1 pistil. In July, a long oval drupe about 1.5 cm long hangs down and ripens red. The crown is in the form of drooping leaves and grows up to 2-3 m in height.

Elaeagnus umbellata Thunberg has many white scale hairs on the twigs, and small fruits less than 1 cm long, and thus is distinguished from Elaeagnus multriflora Thunb. According to "Ik-Sang-Yang-Sul-Dai-Jeon", which is a Korean herbal medicinal book, Elaeagnus multriflora Thunb. is known to be applied generally to treat circulatory diseases and have good effects on poisoning, bruises, stroke, eczema, invigoration, and blood circulation.

Efforts are being made to develop substances that have few side effects and are helpful in preventing and recovering from diseases by studying the efficacy of traditionally known natural products such as Elaeagnus multriflora Thunb., but so far, there is a lack of relevant information on the pharmacological value or physiological activity of Elaeagnus multriflora Thunb.

### Detailed Description of the Invention

### Technical Problem

The present disclosure is to provide a composition for preventing or treating benign prostatic hyperplasia that employs an extract from Elaeagnus multriflora Thunb. fruits, which is a natural raw material, and thus can be safely used without toxicity and side effects.

### Technical Solution

The present disclosure provides include an extract from Elaeagnus multriflora Thunb. fruits as an active ingredient for prevention or treatment of benign prostate hyperplasia, wherein the extract is obtained by solubilizing the fruits in a solvent selected from water, a lower alcohol of 1 to 4 carbon atoms, and a combination thereof.

The pharmaceutical composition for prevention or treatment of benign prostatic hyperplasia according to the present invention may include the extract from Elaeagnus multriflora Thunb. fruits in an amount of 0.1 to 2000 mg/kg/body weight/day, and may also be formulated into powders, granules, tablets, capsules, suspensions, emulsions, syrups, transdermal preparations, suppositories, or sterile injectable solutions.

In the pharmaceutical composition of the present disclosure, the extract from Elaeagnus multriflora Thunb. fruits has an inhibitory activity against the testosterone-induced proliferation of a prostate cancer cell line (LNCaP) or the testosterone-induced weight gain of the prostate gland and the production of prostate-specific antigens (PSA), androgen receptors (AR), and 5α-reductase 2.

### Advantageous Effects

With an extract from Elaeagnus multriflora Thunb. fruits, the composition for the prevention or treatment of BPH according to the present disclosure takes advantage of the domestic natural substance Elaeagnus multriflora Thunb. fruits as a resource and can be used as a pharmaceutical composition or a health functional food composition to prevent or treat BPH safely with neither toxicity nor adverse effects.

### Brief Description of the Drawings

FIG. 1 is a graph showing the inhibitory effect of extracts from Elaeagnus multriflora Thunb. fruits on testosterone-induced proliferation of the prostate epithelial cell line (LNCaP).
FIG. 2 is an image showing the effects on the protein expression of prostate-specific enzyme (5α-reductase 2), androgen receptor (AR), and prostatic specific antigen (PSA) in the testosterone-treated prostate epithelial cell line (LNCaP).
FIG. 3 shows graphs exhibiting inhibitory effects of extracts from Elaeagnus multriflora Thunb. fruits on prostate tissue hyperplasia in rat models with testosterone-induced benign prostate hyperplasia.
FIG. 4 is a graph showing effects of extracts from Elaeagnus multriflora Thunb. fruits on 5-alpha reductase levels in the prostate tissue of rat models with testosterone-induced benign prostate hyperplasia.
FIG. 5 is a graph showing effects of extracts from Elaeagnus multriflora Thunb. fruits on prostatic specific antigen (PSA) levels in the prostate tissue of rat models with testosterone-induced benign prostate hyperplasia.

### Mode for Carrying Out the Invention

The present disclosure is drawn to a composition including an extract from Elaeagnus multriflora Thunb. fruits as an active ingredient for prevention or treatment of benign prostate hyperplasia, and the configuration and effects of the present disclosure will be described in more detail through the following Examples and Comparative Examples.

### 1. Preparation of Extract from Elaeagnus multriflora Thunb.

### 1.1. Preparation of extract from Elaeagnus multriflora Thunb. fruit

An extract from Elaeagnus multriflora Thunb. fruits may be obtained using a solvent selected from water, an lower alcohol of 1 to 4 carbon atoms, and a combination thereof. In brief, 1 liter of distilled water was added to 100 g of Elaeagnus multriflora Thunb. fruits dried through demoisturization, followed by heating at 100°C for 3 hours in a reflux extractor to give an extract. The extract was filtered through a filter (Whatman No. 41) and concentrated in a vacuum.

The concentrated hot-water extract was lyophilized at -50°C for 48 hours in a freeze drier. According to extraction solvents, 12.4 g (12.4%) of an extract from Elaeagnus multriflora Thunb. fruits was obtained and used in the following Experimental Examples.

### 1.2 Preparation of polar and nonpolar solvent-soluble fractions of Elaeagnus multriflora Thunb.

The hot-water extract from Elaeagnus multriflora Thunb. was fractionated using an organic solvent as follows.

### 1.2.1 Isolation of hexane-soluble fraction

In 5 L of distilled water was completely dissolved 12.4 g of the extract from Elaeagnus multriflora Thunb. fruits, and the solution was placed in a fractionation funnel, added with 5 L of hexane to separate a hexane-insoluble layer (aqueous layer) and a hexane-soluble layer. Again, the same procedure was repeated three times for the hexane-insoluble layer (aqueous layer) to collect hexane-insoluble and hexane-soluble fractions.

### 1.2.2 Isolation of chloroform-soluble fraction

The hexane-insoluble fraction (aqueous layer) was mixed with 5 L of chloroform to form chloroform-soluble and chloroform-insoluble fractions. The same procedure was repeated three times for the chloroform-insoluble layer (aqueous layer) to collect chloroform-insoluble and chloroform-soluble fractions.

### 1.2.3 Isolation of ethyl acetate-soluble fraction

The chloroform-insoluble fraction (aqueous layer) was mixed with 5 L of ethyl acetate to form ethyl acetate-soluble and ethyl acetate-insoluble fractions. The same procedure was repeated three times for the ethyl acetate-insoluble layer (aqueous layer) to collect ethyl acetate-insoluble and ethyl acetate-soluble fractions.

### 1.2.4. Isolation of butanol-soluble fraction

The ethyl acetate-insoluble fraction (aqueous layer) was mixed with 5 L of butanol to form butanol-soluble and butanol-insoluble fractions. The same procedure was repeated three times for the butanol-insoluble layer (aqueous layer) to collect butanol-insoluble and butanol-soluble fractions.

### 1.2.5 Isolation of water-soluble fraction

In 5 L of distilled water was completely dissolved 12.4 g of the extract from Elaeagnus multriflora Thunb. fruits, and the hexane-, chloroform-, ethyl acetate-, and butanol-soluble layers were separated by fractionation and then the organic solvent left after concentration was removed to collect the water fraction.

From 12.4 g of the hot-water extract from Elaeagnus multriflora Thunb. fruits, prepared through the hot water extraction and fractionation process, the hexane-, chloroform-, ethyl acetate-, and butanol-soluble fractions were concentrated in a vacuum and lyophilized and used as specimens.

### 2. Assay for cytotoxicity of extract from Elaeagnus multriflora Thunb. fruit

Prior to examining the effect of the extract from Elaeagnus multriflora Thunb. fruits of the present disclosure, a cytotoxicity assay was performed to determine an appropriate concentration capable of producing an effect without exhibiting cytotoxicity.

An extract from Elaeagnus multriflora Thunb. fruits was assayed for cytotoxicity by treating prostate cancer cells with the extract and then measuring the cell viability. The prostate cancer cell line LNCaP cells (the Korean Cell Line Bank 21740) were cultured an RPMI-1640 (Gibco) supplemented with 10% FBS (fetal bovine serum) and 1% penicillin and streptomycin at 37°C under a 5% CO₂ condition.

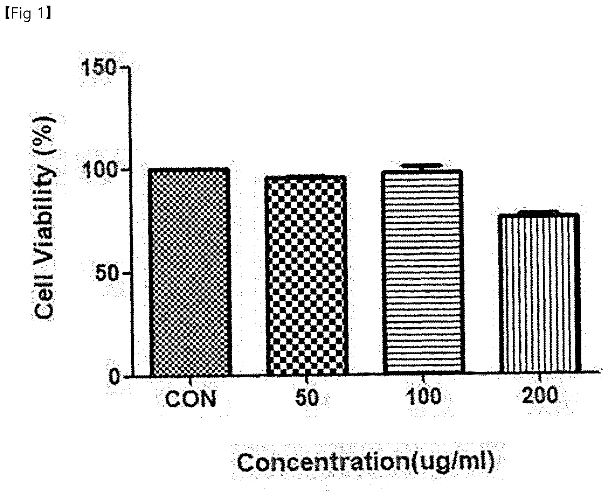

The cells were seeded at a density of 1×10⁵ cells/well into 96-well plates and incubated for 12 hours. Then, the cells were treated with 0, 50, 100, or 200 µg/ml of the extract prepared in Example 1 for 24 hours, followed by adding EZCytox solution in an amount of 10 µl to each well. Absorbance at 450 nm was read using an ELISA reader to assay toxicity. As shown in FIG. 1, the extract from Elaeagnus multriflora Thunb. fruits was found to have no cytotoxicity.

### 3. Assay for inhibitory activity of extract from Stauntonia hexaphylla fruit against expression of PSA, AR, and 5α-reductase 2

Prostate cancer cells were treated with testosterone and an extract from Elaeagnus multriflora Thunb. fruits and then examined for the expression of prostate-specific antigen (PSA), androgen receptor (AR), and prostate-specific enzyme (5α-reductase 2) to determine whether the extract from Elaeagnus multriflora Thunb. fruits is therapeutically effective for benign prostate hyperplasia. LNCaP cells were seeded at a density of 1×10⁵ cells/well into 6-well plates and cultured at 37°C for 24 hours.

Then, the cells were incubated with 1 µM testosterone plus 0, 50, 100, 200 µg/ml of the hot-water extract prepared above for 24 hours before being harvested. For a positive control (BPH), only testosterone was used in an amount of 1 µM, without the extract. A control (Fina) was treated with 10 µM of finasteride, a therapeutic agent for BPH, together with 1 µM of testosterone. A negative control (Control) was not treated with any substance. The cells harvested after incubation was treated with a RIPA buffer (50 mM Tris-HCl, pH 8.0, with 150 mM sodium chloride, 1% NP-40, 0.5% sodium deoxycholate, and 0.1% sodium dodecyl sulfate, with a protease inhibitor cocktail) to separate proteins.

The separated proteins were quantitated using a protein assay kit (Bio-Rad protein assay kit, Bio-Rad, USA). After quantitation, the proteins were run in a 10% polyacrylamide gel by SDS-PAGE and transferred onto a PVDF membrane.

The PVDF membrane was reacted for about 1 hour in 5% skim milk to remove non-specifically binding proteins. Thereafter, the membrane was incubated overnight at 4°C with the primary antibodies anti-AR, anti-PSA, anti-5α-reductase 2, and anti-β-actin antibodies and then for 1 hour at room temperature with a secondary antibody, followed by measuring protein expression levels of AR, PSA, and 5α-reductase 2 with the aid of an ECL kit (Thermo scientific, USA).

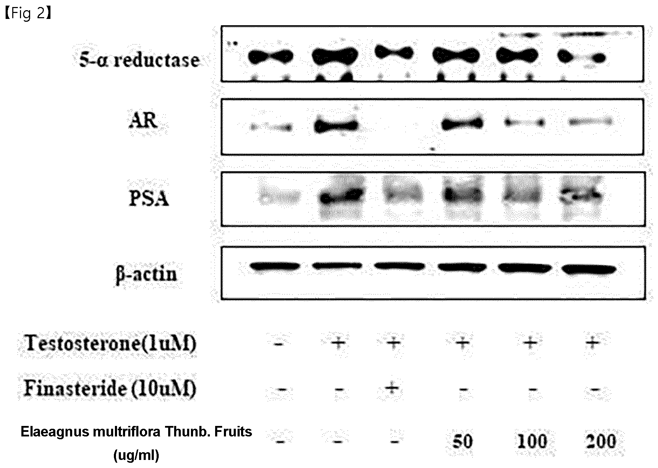

As shown in FIG. 2, remarkable inhibitory effects on the expression of AR and PSA proteins were detected in the cells treated with 100 and 200 µg/ml of the extract from Elaeagnus multriflora Thunb. fruits, compared to the controls. In addition, treatment with 200 ug/ml of the extract from Elaeagnus multriflora Thunb. fruits was observed to inhibit the expression of 5α-reductase 2.

### 4. Construction of benign prostate hyperplasia animal and measurement of prostate gland weight

After being acclimated for one week, male SD rats at nine weeks of age (weighing 330g or less) was subcutaneously injected with a solution of testosterone propionate (TP) in corn oil at a dose of 3 mg/kg for 4 weeks to induce benign prostate hyperplasia.

One hour before injection with TP, the hot-water extract from Elaeagnus multriflora Thunb. fruits, prepared in Section 1., were orally administered at a dose of 50, 100, or 200 mg/kg for four weeks. Serving as a positive control, finasteride (5 mg/kg), which is a 5α-reductase inhibitor used to treat benign prostate hyperplasia, was administered at a dose of 10 mg/kg in the same manner.

The rats were weighed a total of five times at intervals of one week from the start of the experiment and sacrificed the day after the last administration and treatment. The prostate gland was excised from each group (normal group; NC, benign prostate hyperplasia-induced group; BPH, benign prostate hyperplasia-induced + finasteride-administered group; BPH+Fina, benign prostate hyperplasia-induced + extract from Elaeagnus multriflora Thunb. fruits; BPH + extract from Elaeagnus multriflora Thunb. fruit 50, 100, 200mg/kg) and weighed. An inhibitory effect on prostate growth was calculated as follows:

Inhibitory effect on prostate growth (%) = 100 - {(prostate weight of Elaeagnus multriflora Thunb. fruit extract-administered group or positive control - prostate weight of normal group) * 100 / (prostate weight of negative control - prostate group of normal group)}

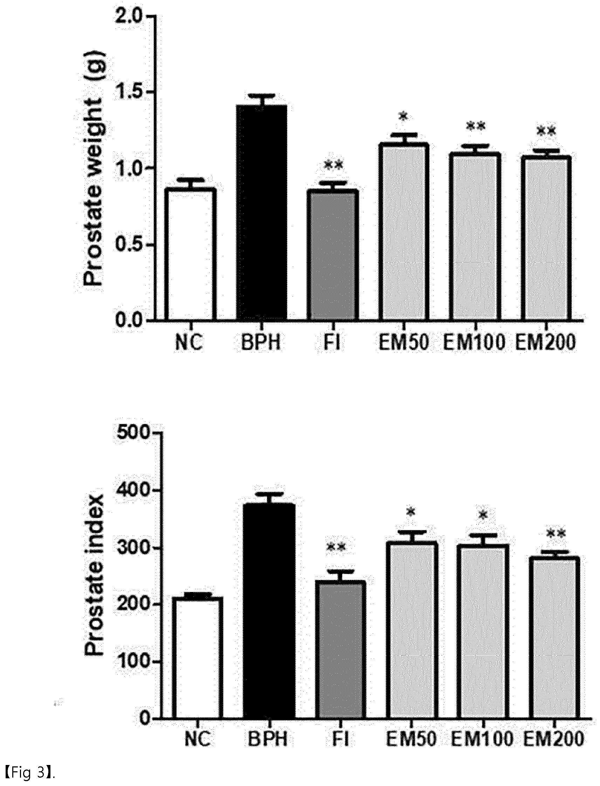

As shown in FIG. 3, the weight of the prostate grand was observed to significantly decrease in the Elaeagnus multriflora Thunb. fruit extract-administered group (BPH+Elaeagnus multriflora Thunb. fruit), compared to the group having TP-induced benign prostate hyperplasia (BPH).

### 5. Measurement of alpha reductase

The prostate tissues of the sacrificed rats were washed once with biological saline and homogenized for one minute with four volumes of phosphate-buffered saline pH 7.4 (Gibco, USA) in a Wise Stir homogenizer (Daihan Scientific, Korea). The prostate tissue homogenate was centrifuged at 5,000 rpm and 4°C for 5 minutes (Micro 17TR, Hanil Science, Korea). The supernatant was isolated and measured for 5-alpha reductase level using a 5-alpha reductase ELISA kit (Mybiosource, USA).

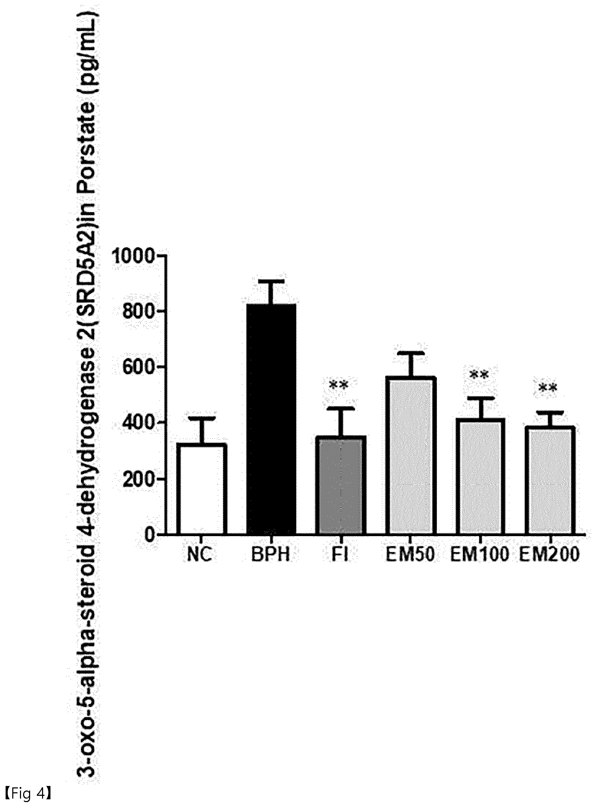

As shown in FIG. 4, treatment with 50, 100, and 200 µg/ml of the extract from Elaeagnus multriflora Thunb. fruits was observed to inhibit the activity of 5-alpha reductase, which plays an essential role in the hypertrophy process of the prostate tissue.

### 6. Measurement of prostatic specific antigen

The prostate tissues of the sacrificed rats were washed once with biological saline and homogenized for one minute with four volumes of phosphate-buffered saline pH 7.4 (Gibco, USA) in a Wise Stir homogenizer (Daihan Scientific, Korea). The prostate tissue homogenate was centrifuged at 5,000 rpm and 4°C for 5 minutes (Micro 17TR, Hanil Science, Korea). The supernatant was isolated and measured for 5-alpha reductase level using a 5-alpha reductase ELISA kit (Mybiosource, USA).

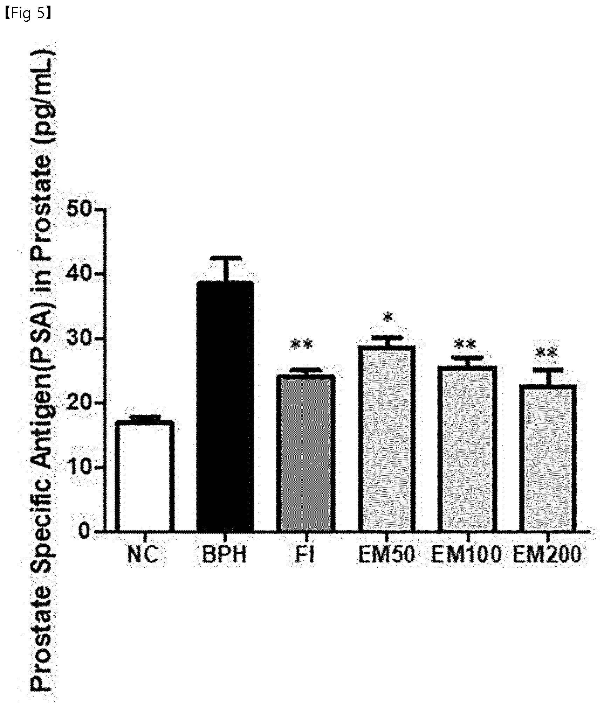

As shown in FIG. 5, treatment with 50, 100, and 200 µg/ml of the extract from Elaeagnus multriflora Thunb. fruits was observed to inhibit the activity of prostatic specific antigen, which increases in prostate tissues, as measured by ELISA.

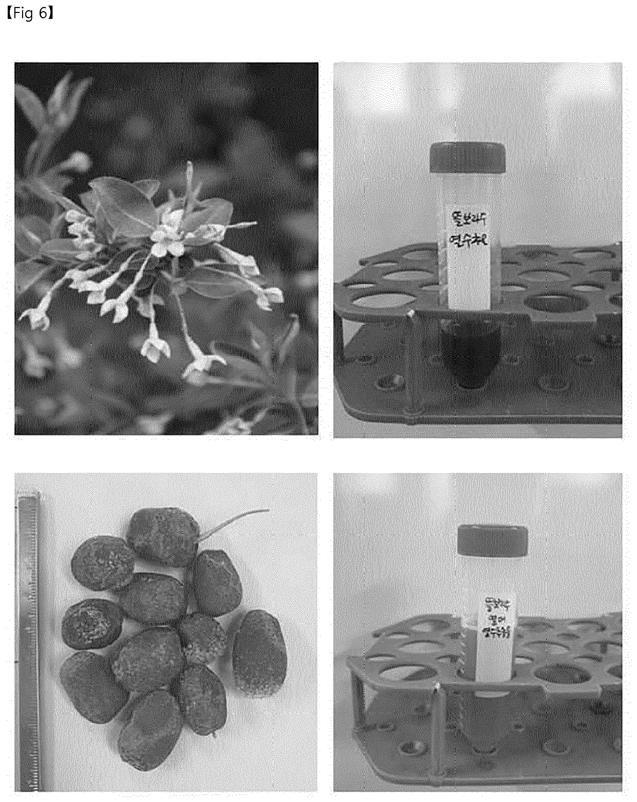

FIG. 6 shows photographic images of Elaeagnus multriflora Thunb. and an extract therefrom according to the present disclosure. Elaeagnus multriflora Thunb. and fruits thereof were heated at 100°C for 3 hours in a reflux extractor. The exudate thus obtained was filtered through a filter and concentrated in a vacuum to afford an extract. The extract can be used in a pharmaceutical composition that can be formulated into pulvis, granules, tablets, capsules, suspensions, emulsions, syrups, transdermal agents, suppositories, or sterile injections, or in a functional food.

### Industrial Applicability

Having excellent prophylactic or preventive effects on benign prostate hyperplasia as proven for testosterone-induced proliferation of a prostate cancer cell line (LNCaP) and in benign prostate hyperplasia rat models, the composition including an extract from Elaeagnus multriflora Thunb. fruits as an active ingredient according to the present disclosure is effective for preventing or inhibiting benign prostate hyperplasia and thus is industrially available.

## Claims

1. A pharmaceutical composition comprising an *Elaeagnus multriflora* Thunb. extract as an active ingredient for prevention or treatment of benign prostatic hyperplasia.

2. The pharmaceutical composition of claim 1, wherein the *Elaeagnus multriflora* Thunb. extract is an extract obtained by solubilization in any one selected from water, an alcohol of 1 to 5 carbon atoms, and a combination thereof.

3. The pharmaceutical composition of claim 1, wherein the pharmaceutical composition is prepared to contain the *Elaeagnus multriflora* Thunb. Extract in an amount of 0.1 to 2000 mg/kg/day.

4. The pharmaceutical composition of claim 2, wherein the pharmaceutical composition is formulated into a pulvis, a granule, a tablet, a capsule, a suspension, an emulsion, a syrup, a transdermal agent, a suppository, or a sterile injection.

5. The pharmaceutical composition of claim 1, wherein the extract has an inhibitory activity against the testosterone-induced proliferation of a prostate (LNCaP) or the testosterone-induced weight gain of the prostate gland and the production of prostate-specific antigens (PSA), androgen receptors (AR), and 5α-reductase 2.

6. A functional food comprising an *Elaeagnus multriflora* Thunb. extract as an active ingredient for prevention or alleviation of benign prostate hyperplasia.
